# EUROPÄISCHE PATENTSCHRIFT

(11) **EP 0 858 447 B1**
(45) Veröffentlichungstag und Bekanntmachung des Hinweises auf die Patenterteilung: **09.01.2002**
(21) Anmeldenummer: 96934767.3
(22) Anmeldetag: 21.10.1996
(51) Int. Cl.: C07C 309/89, C07C 311/39, C07D 239/42, A01N 47/36, C07D 239/46, C07D 239/52, C07D 251/16, C07D 251/46

(54) **VERFAHREN ZUR HERSTELLUNG VON AMINOPHENYLSULFONYLHARNSTOFFEN UND ZWISCHENPRODUKTE ZU DEN VERFAHREN**
METHODS OF PRODUCING AMINOPHENYLSULPHONYL CARBAMIDES AND INTERMEDIATE PRODUCTS FOR USE IN THE PROCESS
PROCEDE DE FABRICATION D'AMINOPHENYLSULFONYL-UREES ET PRODUITS INTERMEDIAIRES POUR CE PROCEDE

(30) Priorität: 02.11.1995 DE 19540701
(43) Veröffentlichungstag der Anmeldung: 19.08.1998
(73) Patentinhaber: Aventis CropScience GmbH, 65929 Frankfurt am Main (DE)
(72) Erfinder: SCHNABEL, Gerhard, D-63868 Grosswallstadt (DE); VERMEHREN, Jan, D-65510 Idstein (DE); WILLMS, Lothar, D-65719 Hofheim (DE)
(86) Internationale Anmeldenummer: EP9604562
(87) Internationale Veröffentlichungsnummer: WO9716419

(56) Entgegenhaltungen:
- EP-A- 0 030 138
- EP-A- 0 330 201
- EP-A- 0 559 044
- DE-A- 4 236 902
- ARCHIV DER PHARMAZIE, Bd. 316, Nr. 5, 1983, WEINHEIM DE, Seiten 464-469, XP000613953 D. MANIA ET AL.: "A new approach to 2-cyanobenzenesulfonamide derivatives"

## Beschreibung

Die Erfindung betrifft das technische Gebiet der Verfahren zur Herstellung von Herbiziden oder Pflanzenwachstumsregulatoren.

Es ist bekannt, daß heterocyclisch substituierte Phenylsulfonylharnstoffe, die am Phenylring eine Amino- bzw. eine funktionalisierte Aminogruppe tragen, herbizide und pflanzenwachstumsregulierende Eigenschaften besitzen (EP-A-1515; EP-A-7687 (= US-A-4,383,113); EP-A-30138 (= US-A-4,394,506); US-A-4,892,946; US-A-4,981,509; EP-A-116518 (= US-A-4,664,695, US-A-4,632,695)), WO-94/10154. Weiterhin wurden in der deutschen Patentanmeldung P 4415049.0 (WO 95/29899) Acylaminosulfonylharnstoffe als Herbizide vorgeschlagen. In der genannten Literatur sind auch Verfahren zur Herstellung der Sulfonylharnstoffe beschrieben. Die Verbindungen mit einer freien Aminogruppe am Phenylring stellen selbst herbizide Wirkstoffe dar oder eignen sich als Ausgangsstoffe zur Herstellung der Verbindungen mit substituierter Aminogruppe.

Die Methoden zur Herstellung der Aminophenylsulfonylharnstoffe sind wegen der vielen reaktiven funktionellen Gruppen im Molekül häufig mit nur geringen Ausbeuten oder geringen Reinheiten möglich. Nachteilig ist auch, daß viele Verfahren auf die Verwendung von Schutzgruppen, z. B. der tert.-Butylgruppe bei Sulfonamiden angewiesen sind, deren Abspaltung spezielle und aufwendig zu handhabende Reagenzien, wie Trifluoressigsäure, erfordern. Außerdem sind die bekannten Verfahren zur Herstellung der Sulfonylharnstoffverbindungen meist vielstufig und ergeben auch deshalb in der Regel nur eine mäßige Gesamtausbeute.

Aufgabe der Erfindung ist somit die Bereitstellung eines Verfahrens, das zur Herstellung einer größeren Gruppe von Herbiziden aus der Reihe der Aminophenylsulfonylharnstoffe geeignet ist, und viele der oben genannten Nachteile vermeidet.

Gegenstand der Erfindung ist ein Verfahren zur Herstellung von Verbindungen der Formel (I) und deren Salzen, 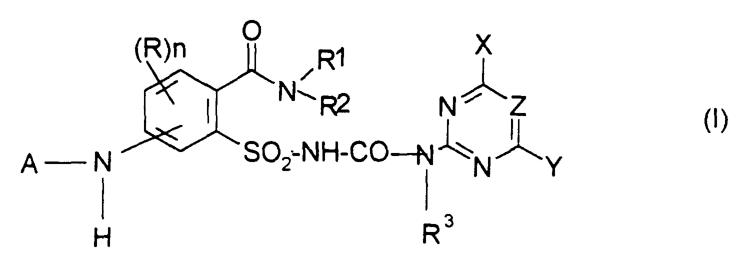 worin
- (R)ₙ: n gleiche oder verschiedene Reste aus der Gruppe Halogen, Alkyl und Alkoxy,
- n: 0, 1, 2 oder 3, vorzugsweise 0 oder 1, insbesondere 0,
- A: Wasserstoff oder einen Acylrest,
- R¹: Wasserstoff oder einen unsubstituierten oder substituierten Kohlenwasserstoff- oder Kohlenwasserstoffoxyrest mit 1 bis 6 C-Atomen, vorzugsweise 1 bis 4 C-Atomen,
- R²: Wasserstoff oder einen unsubstituierten oder substituierten Kohlenwasserstoffrest mit insgesamt 1 bis 10 C-Atomen, vorzugsweise 1 bis 4 C-Atomen,
oder die Gruppe
- NR¹R²: einen heterocyclischen Ring mit 3 bis 8 Ringatomen, der unsubstituiert oder substituiert ist und das N-Atom der Gruppe NR¹R² als Heteroringatom enthält und noch ein oder zwei weitere Heteroringatome aus der Gruppe N, 0 und S enthalten kann,
- R³: Wasserstoff oder C₁-C₄-Alkyl,
- X, Y: unabhängig voneinander Halogen, C₁-C₆-Alkyl, C₁-C₆-Alkoxy, C₁-C₆-Alkylthio, wobei jeder der drei letztgenannten Reste unsubstituiert oder durch einen oder mehrere Reste aus der Gruppe Halogen, C₁-C₄-Alkoxy und C₁-C₄-Alkylthio substituiert ist, oder C₃-C₆-Cycloalkyl, C₂-C₆-Alkenyl, C₂-C₆-Alkinyl, C₃-C₆-Alkenyloxy oder C₃-C₆-Alkinyloxy und
- Z: CH oder N bedeuten,
dadurch gekennzeichnet, daß man
1. (Stufe 1) die Verbindung der Formel (II) 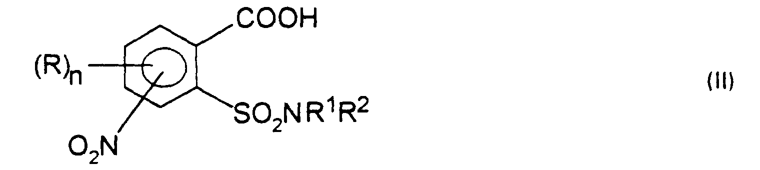 oder deren Salze in einem aprotischen Lösungsmittel in Gegenwart eines Halogenierungsmittels unter Bildung des Carbonsäurehalogenids und dessen Umlagerung zur Verbindung der Formel (III) 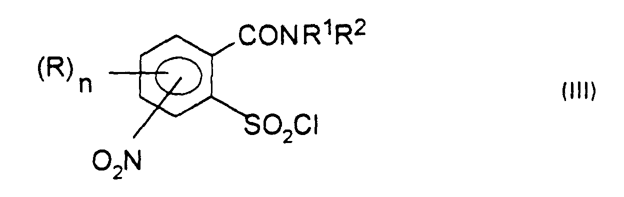 umsetzt,
2. (Stufe 2) anschließend
   a) die Verbindung (III) an der SO₂Cl-Gruppe zur Verbindung der Formel (IV) ammonolysiert, 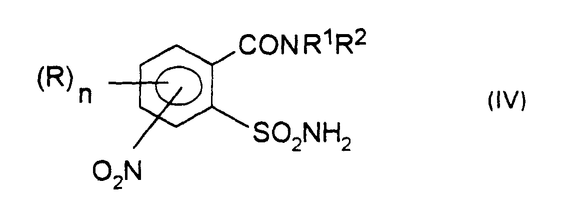 anschließend die Verbindung (IV) an der Nitrogruppe zur Verbindung (V) reduziert 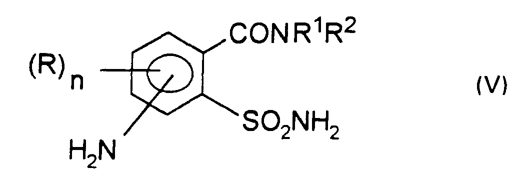 und anschließend die Verbindung (V) mit dem Carbamatsalz der Formel (VI), 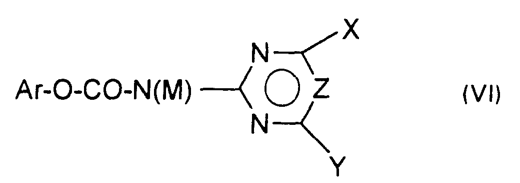 worin Ar ein unsubstituiertes oder substituiertes Phenyl darstellt und M ein Metallkation bedeutet, zur Verbindung der Formel (I) umsetzt, in der R³ in Übereinstimmung mit M in Formel (VI) H oder C₁-C₄-Alkyl oder im Falle M = Metallkation R³ ein Wasserstoffatom bedeutet und A = H bedeutet oder
   b) die Verbindung der Formel (III) an der SO₂Cl-Gruppe zur genannten Verbindung der Formel (IV) ammonolysiert, anschließend die Verbindung (IV) mit dem Carbamat bzw. Carbamatsalz der genannten Formel (VI) zur Verbindung der Formel (VII) umsetzt, 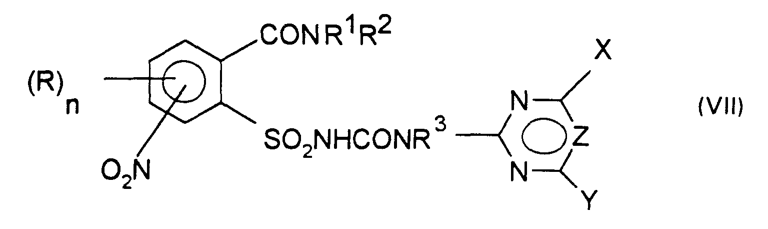 in der R³ in Übereinstimmung mit M in Formel (VI) entweder H oder C₁-C₄-Alkyl oder im Falle M = Metallkation ein Wasserstoffatom bedeutet, und die Verbindung der Formel (VII) an der Nitrogruppe zur Verbindung der Formel (I), worin A = H bedeutet, reduziert oder
   c) die Verbindung der Formel (III) mit Cyanaten und dem heterocyclischen Amin der Formel (VIII). 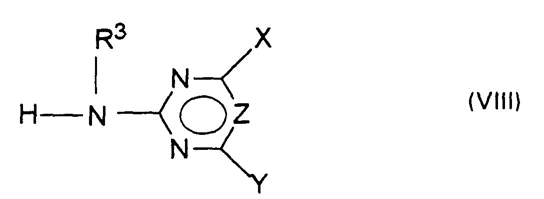 worin R³ wie in Formel (I) definiert ist, zum Sulfonylharnstoff der Formel (VII) umsetzt, welcher durch Reduktion an der Nitrogruppe die Verbindungen der Formel (I), worin A = H bedeutet, ergeben, und
3. (Stufe 3) für den Fall, daß A im Endprodukt der Formel (1) nicht Wasserstoff, sondern einen Acylrest bedeutet, die in Stufe 2 erhaltene Verbindung der Formel (I), worin A = H ist, acyliert,
wobei in den Formeln (II) bis (VIII) die Reste (R)ₙ, R¹, R², X, Y, Z wie im Endprodukt der Formel (I) definiert sind.

In den Formeln (I) bis (VIII) und den im folgenden verwendeten Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten z. B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.
Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z. B. Alkyl, Alkenyl, Alkinyl, Cycloalkyl, Cycloalkenyl oder Aryl, vorzugsweise Alkyl, Alkenyl oder Alkinyl mit bis zu 12 C-Atomen oder Cycloalkyl mit 5 oder 6 Ringatomen oder Phenyl; entsprechendes gilt für einen Kohlenwasserstoffoxyrest.

Ein heterocyclischer Rest oder Ring kann gesättigt, ungesättigt oder heteroaromatisch sein; er enthält ein oder mehrere Heteroringatome, vorzugsweise aus der Gruppe N, O und S; vorzugsweise ist er 5- oder 6-gliedrig und enthält 1, 2 oder 3 Heteroringatome. Der Rest kann z.B. ein wie oben definierter heteroaromatischer Rest oder Ring sein oder ist ein partiell hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heteroaryl, ein substituierter bicyclischer Rest oder Ring oder ein substituierter bicyclischer Rest, gegebenenfalls mit aromatischen Anteilen, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein Acylrest bedeutet den Rest einer organischen Säure, z. B. den Rest einer Carbonsäure und Reste davon abgeleiteter Säuren wie der Thiocarbonsäure, gegebenenfalls N-substituierter Iminocarbonsäuren, oder der Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie (C₁-C₄-Alkyl)-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Von besonderem Interesse sind erfindungsgemäße Verfahren zur Herstellung von Verbindungen der Formel (I) oder deren Salze, worin
- (R)ₙ: n gleiche oder verschiedene Reste aus der Gruppe Halogen, C₁-C₄-Alkyl und C₁-C₄-Alkoxy,
- n: 0 oder 1, insbesondere 0,
- A: H oder Acyl mit 1 bis 8 C-Atomen, insbesondere 1-4 C-Atomen,
- R¹: H, C₁-C₆-Alkyl, C₂-C₆-Alkenyl, C₂-C₆-Alkinyl, C₁-C₆-Alkoxy, C₂-C₆-Alkenoxy, C₂-C₆-Alkinoxy oder C₅-C₆-Cycloalkyl, wobei jeder der 7 letztgenannten Reste unsubstituiert oder durch einen oder mehrere Reste aus der Gruppe Halogen, C₁-C₄-Alkoxy, C₁-C₄-Haloalkoxy, C₁-C₄-Alkylthio, Mono- und Di-(C₁-C₄-Alkyl)-amino, Cyano, Azido, Formyl, (C₁-C₄-Alkyl)-carbonyl, (C₁-C₄-Alkoxy)-carbonyl, C₁-C₄-Alkylsulfinyl und C₁-C₄-Alkylsulfonyl substituiert ist,
oder Phenyl, das unsubstituiert oder durch Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist,
- R²: H, C₁-C₆-Alkyl, C₂-C₆-Alkenyl oder C₂-C₆-Alkinyl, wobei jeder der drei letztgenannten Reste unsubstituiert oder durch einen oder mehrere Reste aus der Gruppe Halogen, C₁-C₄-Alkoxy, C₁-C₄-Haloalkoxy, C₁-C₄-Alkylthio, Mono- und Di-(C₁-C₄-Alkyl)-amino, Cyano, Azido, Formyl, (C₁-C₄-Alkyl)-carbonyl, (C₁-C₄-Alkoxy)-carbonyl, C₁-C₄-Alkylsulfinyl und C₁-C₄-Alkylsulfonyl substituiert ist,
oder die Gruppe
- NR¹R²: einen heterocyclischen Ring aus 4, 5 oder 6 Ringatomen, der bis zu zwei weitere Heteroringatome aus der Gruppe N und O im Ring enthalten kann und der unsubstituiert oder durch einen oder mehrere Reste aus der Gruppe C₁-C₄-Alkyl substituiert ist,
- R³: H oder CH₃,
- einer der Reste X und Y: Halogen, C₁-C₂-Alkyl, C₁-C₂-Alkoxy, C₁-C₂-Alkylthio, wobei jeder der drei letztgenannten Reste unsubstituiert oder durch einen oder mehrere Reste aus der Gruppe Halogen, C₁-C₂-Alkoxy und C₁-C₂-Alkylthio substituiert ist, oder Mono- oder Di(C₁-C₂-alkyl)amino, vorzugsweise Halogen, Methyl oder Methoxy, und
- der andere der Reste X und Y: C₁-C₂-Alkyl, C₁-C₂-Haloalkyl, C₁-C₂-Alkoxy, C₁-C₂-Haloalkoxy oder C₁-C₂-Alkylthio, vorzugsweise Methyl oder Methoxy,
- Z: CH oder N, vorzugsweise CH,
bedeuten.

Bevorzugt sind auch erfindungsgemäße Verfahren zur Herstellung von Verbindungen der Formel (I) oder deren Salzen, worin
- A: Wasserstoff, Formyl, (C₁-C₄)-Alkyl)-carbonyl, das unsubstituiert oder durch einen oder mehrere Reste aus der Gruppe Halogen und (C₁-C₄)-Alkoxy substituiert ist,
oder (C₁-C₄-Alkoxy)-carbonyl, Phenoxycarbonyl, Phenylcarbonyl, Phenyl-(C₁-C₄-alkyl)-carbonyl oder Phenyl-(C₁-C₄-alkoxy)-carbonyl, wobei Phenyl in jedem der letztgenannten 4 Reste unsubstituiert oder substituiert ist, vorzugsweise A Formyl, Acetyl, Propionyl, Methoxycarbonyl, Ethoxycarbonyl, Benzoyl oder Benzyloxycarbonyl, insbesondere Formyl, (C₁-C₄-Alkyl)carbonyl oder (C₁-C₄-Alkoxy)-carbonyl,
bedeutet.

Bevorzugt sind erfindungsgemäße Verfahren zur Herstellung von Verbindungen der Formel (I) oder deren Salze, worin
- R¹: H, C₁-C₂-Alkyl, C₁-C₂-Alkoxy, Phenyl, das unsubstituiert oder durch einen oder mehrere Reste aus der Gruppe Halogen, C₁-C₂-Alkyl, C₁-C₂-Alkoxy, C₁-C₂-Halogenalkyl und C₁-C₂-Halogenalkoxy substituiert ist, insbesondere Methyl oder Ethyl,
- R²: H oder C₁-C₂-Alkyl, insbesondere Methyl oder Ethyl,
oder die Gruppe
- NR¹R²: einen heterocyclischen Ring aus 5 oder 6 Ringatomen, der bis zu einem weiteren Heteroringatom aus der Gruppe N und O im Ring enthalten kann und der unsubstituiert oder durch einen oder mehrere C₁-C₂-Alkylreste substituiert ist, insbesondere Pyrrolidinyl oder Piperidinyl,
- R³: H oder CH₃, insbesondere H,
bedeuten.

Bei den erfindungsgemäßen Verfahren sind solche bevorzugt, bei denen in den Verbindungen der Formel (I) die Gruppe der Formel NH₂ am Phenylrest in para-Stellung zur Gruppe CO-NR¹R² und in meta-Stellung zur SO₂-Gruppe steht.

Gegenstand der Erfindung sind auch die erfinderischen Einzelstufen des Gesamtverfahrens und deren neue Zwischenprodukte insbesondere die 1. Stufe und die Stufen 2a und 2b soweit es die Umsetzung mit Carbamatsalzen (VI), worin M = Kation ist, betrifft.

Die Umsetzung der Verbindungen (II) zu Verbindungen (III) kann mit üblichen Halogenierungsmitteln zur Herstellung von Carbonsäurechloriden, beispielsweise mit Thionylchlorid oder Thionylbromid durchgeführt werden. Dazu läßt man die Nitro-ortho-sulfamoyl-benzoesäure (II) mit einem Überschuß an Halogenierungsmittel in einem aprotischen organischen Lösungsmittel reagieren und erwärmt dann auf eine Temperatur, bei der die Umlagerungsreaktion einsetzt. Als organische Lösungsmittel eignen sich aprotische organische Lösungsmittel, welche gegenüber den Reaktanten inert sind (sogenannte "inerte Lösungsmittel") und deren Siedepunkt über der Temperatur liegt, die für die Umlagerungsreaktion benötigt wird. Die Reaktanten können beispielsweise in homogener Weise oder in heterogener Mischung (z.B. in Suspension) zu den gewünschten Produkten umgesetzt werden. Beispielsweise kann die Umsetzung in gegebenenfalls halogenierten aromatischen Kohlenwasserstoffen wie Toluol, Xylol, Chlorbenzol oder Chlortoluol durchgeführt werden. Die Reaktionstemperaturen für die Halogenierung liegen dabei etwa bei 50 bis 100 °C und für die Umlagerung bei Temperaturen über 100°C bis zum Siedepunkt des aprotischen Lösungsmittels, beispielsweise bei 110 bis 160°C; manchmal findet die Umlagerung in ausreichendem Maße auch schon bei niedrigeren Temperaturen, z.B. bei 70°C statt. Das Thionylchlorid kann beispielsweise äquimolar oder im Überschuß bezogen auf ein Mol der Benzoesäure eingesetzt werden. Anstelle der Benzoesäurederivate können auch die korrespondierenden Salze, wie z.B. die Alkalimetall- oder Erdalkalimetallsalze (z.B. Na-, K-, Li-, Mg- und Ca-Salze), mit einem Halogenierungsmittel, wie z.B. Thionylchlorid, zu den entsprechenden 2-Chlorsulfonylbenzoesäureamid-Derivaten (III) umgesetzt werden.

Es ist bereits bekannt, 2-(N,N-Dialkyl-aminosulfonyl)-benzoesäuren, die unsubstituiert sind oder einfache Alkylgruppen am Phenylring tragen, mit einem 4- bis 8-fachen Überschuß an Thionylchlorid oder Thionylbromid in Benzol, Dichlormethan oder Chloroform zu 2-(Chlorsulfonyl)- bzw. 2-(Bromsulfonyl)-benzoesäure-N,N-dialkylamiden bei Raumtemperatur (25°C) umzusetzen; siehe K. Hovius et al., Tetrahedron Lett. 1983, 3137-3140. Die entsprechende Umsetzung der N,N-Dialkyl-o-sulfamoyl-nitrobenzoesäuren (II) war bisher nicht bekannt und gelang unter diesen Bedingungen nicht. Erst durch Modifikation der bekannten Bedingungen hinsichtlich Temperatur und Lösungsmittel gelingt die Umsetzung auch bei den getesteten Nitrobenzoesäuren (II).

Die benötigten Verbindungen der Formel (II) können auf alternativen Wegen hergestellt werden. So führt die Oxidation der Methylgruppe von Verbindungen der Formel (IX) 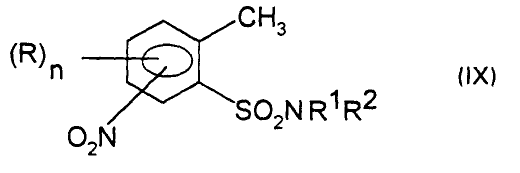 zur Verbindung der Formel (II). Die Oxidation ist beispielsweise analog bekannten Methoden zur Herstellung von Benzoesäuren aus Toluolen durchführbar. Das Toluolderivat der Formel (IX) ist durch Umsetzung des Sulfochlorids der Formel (X) mit einem Amin der Formel HNR¹R² zugänglich;

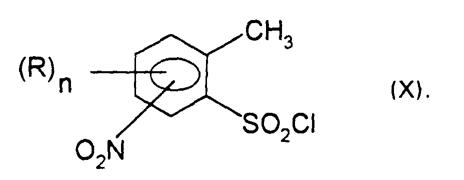

Ein weiterer Zugang zur Verbindung (II) ist die Ammonolyse des Sulfochlorids (XI), 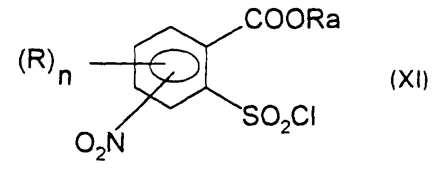 worin Ra einen Alkylrest wie Methyl oder Ethyl bedeutet, mit einem Amin der Formel HNR¹R² zum Sulfonamid und anschließende Verseifung der erhaltenen Verbindung an der Carbonsäureestergruppe. Die einzelnen Umsetzungen können analog bekannten Verfahren gleichen Typs durchgeführt werden. Beispielsweise können die Esterfunktionen mit Alkali- oder Erdalkalihydroxiden wie z.B. LiOH, NaOH, KOH, Mg(OH)₂, Ca(OH)₂ in verschiedenen polaren Solventien, wie z.B. Methanol, Ethanol, Isopropanol, Chlorbenzol, Chlortoluol, Tetrahydrofuran, 1,2-Dimethoxyethan (DME), Diglyme, Dimethylformamid (DMF), N,N-Dimethylacetamid (DMA), N-Methyl-pyrrolidon (NMP) oder Wasser oder Lösungsmittelgemischen aus geeigneten Solventien bei Temperaturen z.B. zwischen -20°C und 150°C, vorzugsweise zwischen -10°C und 100°C, verseift werden.

Die Sulfonamide der Formel (IV) lassen sich durch Umsetzung der Sulfochloride (III) mit Ammoniak (Ammonolyse) in hohen Ausbeuten gewinnen. Die Reaktion läßt sich im allgemeinen bei Temperaturen von -20°C bis 150°C, vorzugsweise von -10°C bis 100°C durchführen. Als Solvens eignen sich unter den Reaktionsbedingungen inerte organische Lösungsmittel, wie z.B.
- dipolar aprotische Lösungsmittel wie DMF, DMA, NMP, Acetonitril,
- Ether wie tert.-Butylmethylether, Dimethoxyethan (DME), THF, Diethylether, Diisopropylether,
- Ester wie Ethylacetat, Butylacetat,
- chlorierte oder unsubstituierte Kohlenwasserstoffe wie Toluol, o-Chlortoluol, Chlorbenzol,
- Alkohole wie Methanol, Ethanol, Isopropanol,
- Wasser, oder
- Gemische aus inerten Solventien.
   Besonders bevorzugte Solventien sind Nitrile wie Acetonitril, Ether wie Diethylether, tert.-Butylmethylether, THF, Dimethoxyethan (DME), oder Ester wie Ethyl- und Butylacetat oder chlorierte oder unsubstituierte Kohlenwasserstoffe wie Toluol, Chlorbenzol oder Chlortoluol.

Die Reduktion der Nitrogruppe in Verbindungen der Formel (IV) kann beispielsweise durch katalytische Hydrierung erfolgen (Stufe 2, Abschnitt a). Für die Hydrierung eignet sich eine Vielzahl von handelsüblichen Katalysatoren, wie z.B. Platin, Palladium oder Raney-Nickel, die analog Standard-Verfahren eingesetzt werden. Als unter den Reaktionsbedingungen inerten organische oder anorganische Lösungsmittel eignen sich beispielsweise
- dipolar aprotische Solventien wie DMA, DMF, NMP oder CH₃CN;
- Ester wie Ethylacetat oder Butylacetat;
- Ether wie DME, Diglyme, Tetraglyme, THF oder Diethylether;
- Alkohole wie Methanol oder Ethanol;
- organische Säuren wie Essigsäure oder Propionsäure;
- Wasser oder
- Gemische von geeigneten inerten Solventien.

Die Reaktionstemperatur kann beispielsweise zwischen -20°C und 150°C, vorzugsweise -10°C bis 100°C, variiert werden. Der Wasserstoffdruck kann ebenfalls in weiten Grenzen variiert werden und beispielsweise von 1 bar bis 200 bar, vorzugsweise von 1 bar bis 100 bar, insbesondere von 1 bar bis 50 bar betragen.

Die Umsetzung von Verbindungen (V) mit Carbamaten der Formel (VI) soll vorzugsweise weitgehend selektiv an der Sulfonamidgruppe anstatt an der alternativen Aminogruppen am Phenylrest erfolgen. Wird die Verbindung (V) mit Carbamaten der Formel (VI) nach Standardverfahren durchgeführt, z.B. in Acetonitril in Gegenwart einer sterisch gehinderten Base wie 1,8-Diazabicyclo[5.4.0]undec-7-en (DBU), so sind Sulfonamid- und Aminofunktion ähnlich reaktiv, d.h. die Chemoselektivität der Reaktion ist völlig unbefriedigend. So führt beispielsweise die Umsetzung von 5-Amino-2-dimethylaminocarbonyl-benzolsulfonamid mit 4,6-Dimethoxy-2-phenoxycarbonylamino-pyrimidin in Gegenwart von DBU zu zwei Produkten im Verhältnis 2:1, nämlich zu 5-Amino-2-dimethylaminocarbonyl-N-[(4,6-dimethoxypyrimidin-2-yl)-aminocarbonyl]-benzolsulfonamid und 5-[(4,6-Dimethoxypyrimidin-2-yl)-aminocarbonylamino]-2-dimethylaminocarbonyl-N-[(4,6-dimethoxypyrimidin-2-yl)aminocarbonyl]-benzolsulfonamid (Nebenprodukt) (siehe Vergleichsbeispiel).

Die Umsetzung kann jedoch überraschend selektiv im Falle des Umsatzes mit Carbamatsalzen durchgeführt werden. Erfindungsgemäß werden z.B. im Vergleich zur Umsetzung mit Carbamaten der Formel (XI), worin M = Wasserstoff bedeutet, bessere Selektivitäten erreicht, wenn Carbamatsalze (VI), worin M = Kation bedeutet, z.B. Natrium- oder Kaliumsalze der Carbamate, eingesetzt werden.
Hierzu wird beispielsweise zunächst das Carbamat (VI) (M=H) mit geeigneten Basen, z.B. Alkali- und Erdalkalihydroxiden wie LiOH, NaOH, KOH, Mg(OH)₂, Ca(OH)₂ oder Tetralkylammoniumhydroxiden wie Tetramethylammoniumhydroxid oder Alkali- oder Erdalkalihydriden wie z.B. NaH, KH, CaH₂ oder Alkali- oder Erdalkalialkoholaten wie NaOCH₃, NaOC₂H₅, Natrium-isopropylat, Natrium-tert.-butylat, KOCH₃, KOC₂H₅, Kalium-isopropylat, Kalium-tert-butylat oder Mischungen von Basen in geeigneten Solventien zu einem Salz der Formel (VI) (M = Kation) umgesetzt.
Als Solventien eignen sich inerte organische Lösemittel, vorzugsweise aprotische unpolare oder aprotische polare Lösungsmittel wie Ether (z.B. THF, DME, Diethylether, Diisopropylether, Dioxan, tert.-Butylmethylether), Amide (z.B. DMF, DMA, NMP), gegebenenfalls halogenierte aromatische Kohlenwasserstoffe wie Toluol, Chlortoluol oder Chlorbenzol.
Für Alkali- und Erdalkalisalze sind weiterhin auch Alkohole wie z.B. Methanol, Ethanol, Isopropanol oder Solvensmischungen geeigneten Solventien.
Für Alkali-, Erdalkali- und Tetraalkylammoniumhydroxide stellt zudem auch Wasser ein geeignetes Solvens oder eine Solvenskomponente dar.
Besonders bevorzugt sind Ether wie THF, DME oder Dioxan.

Die so erzeugten Salze der Formel (VI) (M = Kation) werden vorteilhaft in Lösung erzeugt und ohne Zwischenisolierung für die weitere Umsetzung eingesetzt. Hierzu werden beispielsweise die Salze in Lösung mit Sulfonamiden der Formel (V) bei Temperaturen von -40°C bis 150°C, vorzugsweise von -40°C bis 80°C, insbesondere von -20°C bis 80°C, umgesetzt. Nach dem Ansäuern der Reaktionslösung mit Säuren z.B. organischen Säuren wie Ameisen- oder Essigsäure oder Mineralsäuren wie Salzsäure oder Schwefelsäure, können die Sulfonylharnstoffe der Formel (I) (A=H) (= Verbindungen (I')) nach Standard-Methoden isoliert werden. Eine günstige Ausbeute wird in der Regel bei einem Molverhältnis von Sulfonamiden (V) zu Carbamat-Salz (VI) von 1:0,7 bis 1:1,5 erreicht.

Bevorzugte Varianten sind die Umsetzungen von Carbamaten der Formel (VI) (M=H) mit Alkalimetallhydroxiden oder -alkoholaten, wie z.B. KOH, NaOCH₃, KOCH₃, Natrium- oder Kalium-tert-butylat, Natrium- oder Kalium-isopropylat, insbesondere mit den sterisch anspruchsvollen Alkalialkoholaten, in dipolaren aprotischen Solventien wie THF, Dioxan, DMF, DMA, insbesondere THF, DME oder Dioxan.

Einen alternativen Zugang zu Sulfonylharnstoffen (I') (= Formel (I) mit A = H) bietet die genannte Variante 2b. Danach werden aus den Verbindungen (III) zunächst wie im 1. Schritt in Stufe 2a) durch Ammonolyse Nitrobenzolsulfonamide der Formel (IV) erhalten, welche anschließend mit Carbamaten der Formel (VI) analog Standardbedingungen in Gegenwart von Basen, z.B. organischen Stickstoffbasen wie DBU oder Triethylamin, Alkali- oder Erdalkalihydroxiden wie LiOH, NaOH, KOH, Mg(OH)₂ oder Ca(OH)₂, Alkali- oder Erdalkalialkoholaten wie z.B. NaOCH₃, KOCH₃, Na- oder K-isopropylat, Na- oder K-tert-Butylat, zu den Nitrosulfonylharnstoffen der Formel (VII) umgesetzt werden. Die Verhältnisse der Reaktanten sind vorzugsweise von 0,7 bis 1,5 Äquivalenten Carbamate (VI) und 0,7 bis 2,2 Äquivalenten Base, jeweils bezogen auf 1 Äquivalent Sulfonamid (IV).

Die Umsetzungen der Verbindungen (IV) zu Verbindungen (VII) erfolgen bei Temperaturen von beispielsweise -20°C bis 100°C, vorzugsweise von -10°C bis ca. 70°C, in inerten organischen Solventien, z.B. in aprotischen Solventien wie Ethern (z.B. THF, DME, Dioxan, Diethylether), Acetonitril, DMF, DMA, NMP, Alkoholen, Estern wie Ethylacetat oder Butylacetat, chlorierten aliphatischen oder aromatischen Kohlenwasserstoffen wie Dichlormethan, Trichlorethan, Chlorbenzol oder o-Chlortoluol oder protischen Solventien wie z.B. Methanol, Ethanol, Isopropanol oder Wasser oder entsprechenden Solvensgemischen.

Die Nitrosulfonylharnstoffe der Formel (VII) lassen sich auch analog der oben beschriebenen Umsetzung von Carbamat-Salzen der Formel (VI) (M = Kation) mit Sulfonamiden der Formel (V) zu den Sulfonylharnstoffen (I') (=Formel (I) mit A=H) durch Reaktion von Sulfonamiden der Formel (IV) (M = Kation) mit Carbamat-Salzen (M = Kation) (VI) gewinnen.

Anschließend lassen sich die Nitrosulfonylharnstoffe (VII) durch katalytische Hydrierung zu den Aminosulfonylharnstoffen (I') der Formel (I), worin A=H ist, umsetzen.
Die Hydrierung kann analog der oben beschriebenen Hydrierung von Verbindungen der Formel (IV) gemäß Standard-Verfahren durchgeführt werden. Beim Einsatz eines wäßrigen Mediums als Lösungsmittel sind basische Lösungen oder gepufferte wäßrige Lösungen mit einem pH von = 5 bis 13, vorzugsweise von 7 bis 11, besonders geeignet.
Alternativ können anstelle der neutralen Nitrosulfonylharnstoffe (VII) deren Salze für die Hydrierungen eingesetzt werden.
Geeignete Kationen zu den Sulfonylharnstoffanionen der Formel (VII) sind z.B. Alkali- oder Erdalkalikationen, wie z.B. Li⁺, Na⁺, K⁺, Cs⁺, Mg²⁺, Ca²⁺, oder Ammoniumkationen wie z.B. NH₄⁺, HN(CH₃)₃⁺, N(CH₃)₄⁺, N(C₂H₅)₄⁺, HN(C₂H₅)₃⁺, (DBU-H)⁺ oder Gemische dieser Kationen.

Die Methode der Umsetzung des Sulfochlorids (III) mit Cyanaten, wie z.B. Natriumcyanat oder Kaliumcyanat, und heterocyclischen Aminen (VIII) zu Verbindungen (VII) (Variante 2c) ist im Prinzip in der obengenannten Literatur beschrieben, siehe z.B. deutsche Patentanmeldung P 4415049.0 (WO 95/29899). Die nachfolgende Reduktion der Nitrogruppe kann wie im Falle der Verbindung (IV) nach üblichen Methoden, z.B. vorzugsweise durch katalytische Reduktion erfolgen, wie sie bereits oben für Verbindungen (VII) beschrieben ist.

In der 3. Stufe können die aus der 2. Stufe erhaltenen Verbindungen (I') der Formel (I), in denen A = H ist, zu herbiziden Wirkstoffen (I") der Formel (I), worin A = Acylrest bedeutet, acyliert werden. Die Acylierung gelingt überraschenderweise sehr selektiv an der Aminogruppe, die am Phenylring gebunden ist, mit üblichen Acylierungsmittel. Die Acylierung wird z. B. in einem aprotischen organischen Lösungsmittel durchgeführt. Die Acylierung kann mit üblichen Acylierungsreagenzien erfolgen. Beispiele für Acylierungsmittel sind Anhydride, Carbonsäurehalogenide, aktivierte Ester (= Aktivester), wie Kohlensäureester und Chlorkohlensäureester, Sulfonsäurechloride etc. Beispielsweise stehen für die Formylierung der Aminofunktion von Sulfonylharnstoffen (I') der Formel (I), worin A=H ist, eine Reihe von sehr guten Standard-Verfahren zur Verfügung. So läßt sich die Aminofunktion mit gemischten Anhydriden der Formel (XII)

H-CO-O-CO-R (XII)

R = Alkyl
oder mit Ameisensäure in die Formylaminogruppe überführen.

Gemischte Anhydride können nach literaturbekannten Verfahren aus Ameisensäure und Carbonsäureanhydriden wie z.B. Acetanhydrid oder aus Ameisensäuresalzen, wie z.B. Natriumformiat und Carbonsäurechloriden, wie z.B. Acetylchlorid oder Pivaloylchlorid, hergestellt werden.

Die einzelnen Zwischenprodukte des erfindungsgemäßen Verfahrens sind neu und sind ebenfalls Gegenstand der Erfindung.

Die einzelnen Reaktionsstufen des beschriebenen Gesamtverfahrens können in homogener, auch in übersättigter, d.h. kinetisch stabiler, Lösung oder in heterogenen Suspensionen durchgeführt werden, um jeweils vorteilhafte Raum-Zeit-Ausbeuten zu erzielen. Dabei werden im allgemeinen sehr gute Ausbeuten und Reinheiten erzielt. Durch geschickte Kombination der Einzelstufen lassen sich Gesamtausbeuten an Sulfonylharnstoffen der Formel (I), vorzugsweise solchen mit der Acylaminofunktion in para-Stellung zur Carbonamidgruppe, erzielen, die den in der Literatur (DE 4415049, WO 95/29899) beschriebenen Ausbeuten überlegen sind.

Durch geschickte Wahl der Reaktionsbedingungen lassen sich zudem mehrere Stufen zu Eintopf- bzw. Kaskaden-Reaktionen zusammenfassen. Hierdurch läßt sich die Ausbeute als auch die Raum-Zeit-Ausbeute teilweise erheblich verbessern.

Bevorzugt ist die Herstellung von Verbindungen der Formel (I), worin n = 0 und R³ = H bedeuten und die Aminogruppe am Phenylrest in Parastellung zur Carbonamidgruppe und in Metastellung zur SO₂-Funktion steht (= Verbindungen (la)).
Besonders bevorzugt ist das erfindungsgemäße Verfahren und deren Teilstufen, dadurch gekennzeichnet, daß man eine Verbindung der Formel (lla) 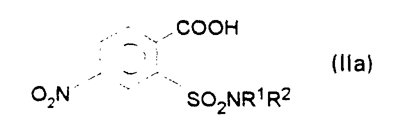 worin R¹ und R² wie in Formel (I) definiert sind, in Gegenwart eines Halogenierungsmittels unter Säurehalogenidbildung und Umlagerung zur Verbindung der Formel (IIIa) umsetzt, 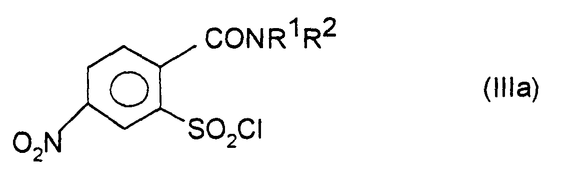 anschließend die Verbindung (IIIa) mit Ammoniak in das Amid der Formel (IVa) überführt 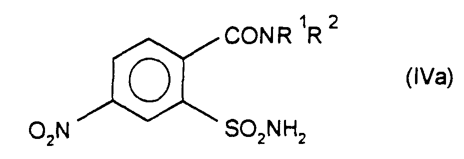 und anschließend die Verbindung (IVa) an der Nitrogruppe zur Verbindung (Va) reduziert, 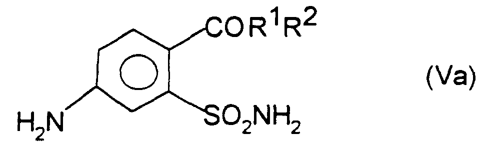 und die Verbindung (Va) mit Carbamat-Salz der Formel (VIa) 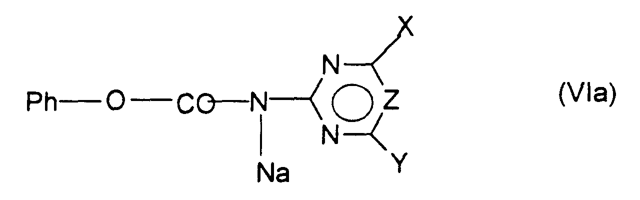 zur Verbindung (la) umsetzt.

In den folgenden Herstellungsbeispielen beziehen sich Mengen und Prozentangaben auf das Gewicht, sofern nichts anderes angegeben ist. Die Abkürzung "Fp" steht für "Schmelzpunkt".

Herstellungsbeispiele

### Beispiel 1

### 2-Dimethylaminocarbonyl-5-nitrobenzolsulfonsäurechlorid

Zu einer Suspension aus 195,2 g 2-Carboxy-5-nitrobenzolsulfonsäuredimethylamid in 800 ml Chlorbenzol werden 40 ml Thionylchlorid zugesetzt. Anschließend wird das Gemisch unter kräftigem Rühren langsam auf 70 bis 75°C erhitzt. Nach der Zugabe von weiteren 120 ml Thionylchlorid wird das Reaktionsgemisch bis zum Sieden erhitzt. Nach beendeter Reaktion wird das Reaktionsgemisch unter reduziertem Druck eingeengt. Man erhält so 209 g des gewünschten Produktes, das für weitere Umsetzungen genügend rein ist, Fp.: 129-131°C.

### Beispiel 2

### 2-Dimethylaminocarbonyl-5-nitrobenzolsulfonsäureamid

Bei einem Gemisch aus 77,8 g 2-Dimethylaminocarbonyl-5-nitrobenzolsulfonsäurechlorid und 780 ml Tetrahydrofuran werden bei 5°C unter Rühren 37 ml konzentrierte Ammoniaklösung (33 %ig) zugetropft. Das Gemisch wird bis zum Ende der Reaktion nachgerührt. Das Reaktionsgemisch wird unter reduziertem Druck eingeengt, und der Rückstand wird mit wenig Wasser verrührt. Nach dem Trocknen erhält man 66,9 g des gewünschten Produktes.
Fp.: 159-160°C.

### Beispiel 3

### 5-Amino-2-dimethylaminocarbonylbenzolsulfonsäureamid

Eine Lösung aus 12,5 g 2-Dimethylaminocarbonyl-5-nitrobenzolsulfonamid in 250 ml Methanol wird mit 1 g feuchtem Raney-Nickel versetzt und bei 60°C und 50 bar Wasserstoffdruck kräftig durchmischt. Nach beendeter Wasserstoffaufnahme wird der Katalysator abgetrennt und das Filtrat eingeengt. Man erhält so 11,0 g an gewünschtem Produkt.

### Beispiel 4

### 5-Amino-2-dimethylaminocarbonyl-N-[(4,6-dimethoxypyrimidin-2-yl)-aminocarbonyl]-benzolsulfonamid

### Methode A:

128,4 g 4,6-Dimethoxy-2-(phenoxycarbonylamino)pyrimidin werden bei 0°C in 1250 ml THF (Tetrahydrofuran) vorgelegt. Nach der Zugabe von 44,8 g Natrium-tert.-butylat wird diese Lösung zu einem Gemisch aus 108,1 g 5-Amino-2-dimethylamino-carbonylbenzolsulfonamid in 1250 ml THF bei 0-2°C zugetropft. Nach beendeter Reaktion wird das Reaktionsgemisch eingeengt. Der Rückstand wird zwischen 1500 ml Wasser und 780 ml Petrolether verteilt und mit konzentrierter Salzsäure vorsichtig angesäuert (100 ml). Der ausgefallene Feststoff wird mit Petrolether und Essigsäureethylester gewaschen. Nach dem Trocknen werden 186,9 g des gewünschten Produktes erhalten.
Fp.: 192-193°C unter Zers.

### Methode B:

a) Zu einer Suspension aus 3,78 g Natriumcyanat, 4,7 ml Pyridin und 100 ml Acetonitril werden nacheinander 5,3 g 2-Amino-4,6-dimethoxypyrimidin und 10 g 2-Dimethylaminocarbonyl-5-nitrobenzolsulfonsäurechlorid zugegeben. Das Gemisch wird bei Raumtemperatur bis zur vollständigen Umsetzung nachgerührt und anschließend in verdünnte, gekühlte Salzsäure eingetragen. Das angefallene Rohprodukt wird mittels Säulenchromatographie (CH₂Cl₂/CH₃OH = 95/5) gereinigt. Man erhält so 3,1 g des gewünschten Produktes; Fp.: 182-186°C unter Zers.
b) 1,4 g 2-Dimethylaminocarbonyl-5-nitro-N-[(4,6-dimethoxypyrimidin-2-yl)amino-carbonyl]-benzolsulfonamid werden in 25 ml Wasser suspendiert und mit 5,5 ml 1 N Natronlauge versetzt. Nach der Zugabe von 0,1 g Palladium auf Kohle (10 %ig, 50 % Wasser) wird das Gemisch bei Raumtemperatur unter einer Wasserstoffatmosphäre (1 bar) kräftig gerührt. Nach beendeter Reaktion wird der Katalysator durch Filtration abgetrennt und mit wenig Wasser gewaschen. Nach dem Ansäuern der wässrigen Phase mit konzentrierter Salzsäure werden 1,1 g des gewünschten Produktes erhalten; Fp.: 192-193°C unter Zers.

### Beispiel 5

### N,N-Dimethyl-2-methoxycarbonyl-5-nitrobenzolsulfonamid

Zu einem Gemisch aus 202,8 g 2-Methoxycarbonyl-5-nitrobenzolsulfochlorid und 65,1 g Dimethylaminhydrochlorid in 1000 ml Acetonitril werden bei 5°C unter kräftigem Rühren 250,6 g Kaliumcarbonat zugesetzt. Nach beendeter Reaktion wird der Feststoff mittels Filtration abgetrennt und mit Ethylacetat nachgewaschen. Die vereinigten organischen Phasen werden anschließend unter reduziertem Druck eingeengt. Man erhält so 206,7 g des gewünschten Produktes. Fp.: 93-96°C

### Beispiel 6

### N,N-Dimethyl-2-carboxy-5-nitrobenzolsulfonamid

Zu einer Lösung aus 206,7 g 2-Methoxycarbonyl-5-nitrobenzolsulfonsäuredimethylamid in 1500 ml Methanol werden 60,2 g Lithiumhydroxidmonohydrat zugesetzt. Das Gemisch wird anschließend bis zum vollständigem Umsatz bei 50°C gerührt. Nach dem Einengen des Reaktionsgemisches unter reduziertem Druck wird der Rückstand in Wasser aufgenommen und bei 0°C mit konzentrierter Salzsäure versetzt (pH = 1). Nach dem Absaugen und Trocknen wird die gewünschte Säure erhalten. Ausbeute: 162,9 g; Fp.: 160-163°C

### Beispiel 7

### 2-Dimethylaminocarbonyl-5-nitro-N-[(4,6-dimethoxypyrimidin-2-yl)-aminocarbonyl]-benzolsulfonamid

### 1. Methode:

31,7 g 4,6-Dimethoxy-2-(phenoxycarbonylamino)-pyrimidin werden bei 0°C in 400 ml THF vorgelegt. Nach der Zugabe von 11,08 g Natrium-tert.-butylat wird diese Lösung zu einem Gemisch aus 30,0 g 2-Dimethylaminocarbonyl-5-nitrobenzolsulfonsäureamid in 400 ml THF bei 0°C zugetropft. Nach beendeter Reaktion wird das Reaktionsgemisch eingeengt, zwischen 500 ml Wasser und 250 ml Petrolether verteilt und mit konzentrierter Salzsäure angesäuert. Der angefallene Feststoff wird mit Petrolether und Essigsäureethylester gewaschen. Nach dem Trocknen werden 43,4 g des gewünschten Produktes erhalten: Fp.: 182-186°C unter Zersetzung.

### 2. Methode:

Zu einer Suspension aus 5,0 g 2-Dimethylaminocarbonyl-5-nitrobenzolsulfonamid in 20 ml Wasser werden bei Raumtemperatur und unter kräftigem Rühren 18,6 ml 1N Natronlauge zugetropft. Anschließend werden 5,04 g 4,6-Dimethoxy-2-phenoxycarbonylamino-pyrimidin zugesetzt. Das Reaktionsgemisch wird auf ca. 50 bis 60°C erwärmt und bis zum vollständigen Umsatz bei dieser Temperatur gerührt. Die wäßrige Phase wird nun mit Diisopropylether gewaschen. Die wäßrige Phase wird mit konzentrierter Salzsäure angesäuert (pH = 2 bis 3). Der ausgefallene Feststoff wird abgetrennt, mit Wasser gewaschen und getrocknet. Man erhält so 7,6 g an gewünschtem Produkt, das eine ausreichende Reinheit für weitere Umsetzungen besitzt.

### Beispiel 8

### N-[(4,6-Dimethoxypyrimidin-2-yl)amino-carbonyl]-2-dimethylaminocarbonyl-5-acetylaminobenzolsulfonamid

Zu einer Mischung aus 0,64 g N-[(4,6-Dimethoxypyrimidin-2-yl)aminocarbonyl]-5-amino-2-dimethylaminocarbonylbenzolsulfonamid und 10 ml Dimethylacetamid werden langsam 0,13 ml Acetylchlorid zugetropft. Nach beendeter Reaktion wird das Reaktionsgemisch unter reduziertem Druck eingeengt, und der Rückstand wird mit Wasser und Essigsäureethylester gewaschen. Man erhält so 0,45 g des gewünschten Produktes in hoher Reinheit (> 92 %, HPLC).

### Beispiel 9

### N-[(4,6-Dimethoxypyrimidin-2-yl)-aminocarbonyl]-5-formylamino-2-dimethylaminocarbonylbenzolsulfonamid

Zu dem gemischten Anhydrid, das in situ aus 0,5 ml Ameisensäure und 1,0 ml Essigsäureanhydrid nach Standardverfahren hergestellt wurde, wird eine Lösung aus 1,9 g N-[(4,6-Dimethoxypyrimidin-2-yl)-aminocarbonyl]-5-amino-2-dimethylaminocarbonylbenzolsulfonamid und 10 ml Dichlormethan zugetropft. Nach beendeter Reaktion wird das Reaktionsgemisch eingeengt und der Rückstand mit Wasser und Ethylacetat gewaschen. Es werden so 1,8 g an gewünschtem Produkt gewonnen; Reinheit > 92 % (HPLC).

### Beispiel 10

### N-[(4,6-Dimethoxypyrimidin-2-yl)-aminocarbonyl]-2-dimethylaminocarbonyl-5-propionylaminobenzolsulfonamid

Zu einer Lösung aus 0,64 g 5-Amino-N-[(4,6-Dimethoxypyrimidin-2-yl)-aminocarbonyl]-2-dimethylaminocarbonyl-benzolsulfonamid in 10 ml Dimethylacetamid werden langsam 0,13 ml Propionylchlorid zugetropft. Nach beendeter Reaktion wird das Reaktionsgemisch unter reduziertem Druck eingeengt, und der Rückstand wird mit Wasser und Essigsäureethylester gewaschen. Man erhält so 0,45 g des gewünschten Produktes in hoher Reinheit (> 92 %, HPLC).

### Beispiel 11 (Vergleichsbeispiel)

### 5-Amino-2-dimethylaminocarbonyl-N-[(4,6-dimethoxypyrimidin-2-yl)-aminocarbonyl]-benzolsulfonamid

Zu einer Suspension aus 1,0 g 5-Amino-2-dimethylaminocarbonylbenzolsulfonamid und 1,1 g 4,6-Dimethoxy-2-(phenoxycarbonylamino)-pyrimidin in 10 ml Acetonitril werden bei 0°C 0,6 ml 1,8-Diazabicyclo[5.4.0]undec-7-en (DBU) unter Rühren zugesetzt. Das Gemisch wird bis zur vollständigen Umsetzung nachgerührt. Nach dem Abdestillieren der flüchtigen Komponenten wird der Rückstand in wenig Wasser aufgenommen und mit Diethylether gewaschen. Die wäßrige Phase wird anschließend mit konzentrierter Salzsäure angesäuert (pH = 2-3). Der abgeschiedene Feststoff wird mit Diisopropylether gewaschen und anschließend getrocknet. Man erhält so 1,4 g eines Feststoffes, der die beiden Verbindungen 5-Amino-2-dimethylaminocarbonylN-[(4,6-dimethoxypyrimidin-2-yl)-aminocarbonyl]-benzolsulfonamid und 5-[(4,6-Dimethoxypyrimidin-2-yl)aminocarbonylamino]-2-dimethylaminocarbonyl-N-[(4,6-dimethoxypyrimidin-2-yl)aminocarbonyl]-benzolsulfonamid im Verhältnis von ca. 2:1 enthält.

## Patentansprüche

1. Verfahren zur Herstellung einer Verbindung der Formel (I) und deren Salzen, 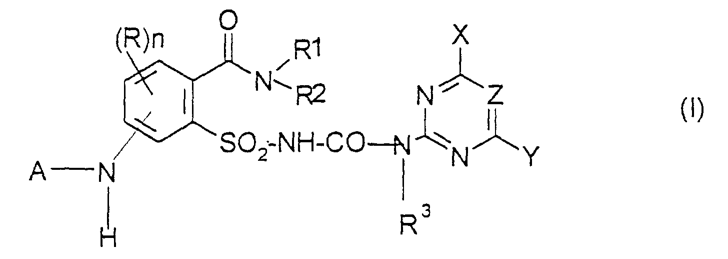 worin
(R)ₙ n gleiche oder verschiedene Reste aus der Gruppe Halogen, Alkyl und Alkoxy,
n 0, 1, 2 oder 3,
A Wasserstoff oder einen Acylrest,
R¹ Wasserstoff oder einen unsubstituierten oder substituierten Kohlenwasserstoff- oder Kohlenwasserstoffoxyrest mit 1 bis 6 C-Atomen,
R² Wasserstoff oder einen unsubstituierten oder substituierten Kohlenwasserstoffrest mit insgesamt 1 bis 10 C-Atomen,
oder die Gruppe
NR¹R² einen heterocyclischen Ring mit 3 bis 8 Ringatomen, der unsubstituiert oder substituiert ist und das N-Atom der Gruppe NR¹R² als Heteroringatom enthält und noch ein oder zwei weitere Heteroringatome aus der Gruppe N, O und S enthalten kann,
R³ Wasserstoff oder C₁-C₄-Alkyl,
X, Y unabhängig voneinander Halogen, C₁-C₆-Alkyl, C₁-C₆-Alkoxy, C₁-C₆-Alkylthio, wobei jeder der drei letztgenannten Reste unsubstituiert oder durch einen oder mehrere Reste aus der Gruppe Halogen, C₁-C₄-Alkoxy und C₁-C₄-Alkylthio substituiert ist, oder C₃-C₆-Cycloalkyl, C₂-C₆-Alkenyl, C₂-C₆-Alkinyl, C₃-C₆-Alkenyloxy oder C₃-C₆-Alkinyloxy und
Z CH oder N bedeuten,
**dadurch gekennzeichnet, daß** man
1. die Verbindung der Formel (II) 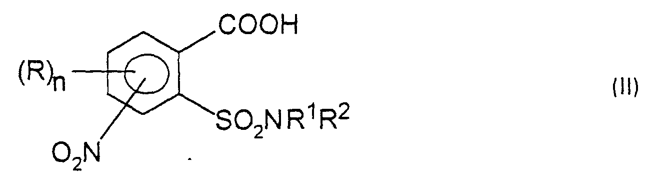 oder deren Salze in einem aprotischen Lösungsmittel in Gegenwart eines Halogenierungsmittels unter Bildung des Carbonsäurehalogenids und dessen Umlagerung zur Verbindung der Formel (III) umsetzt, 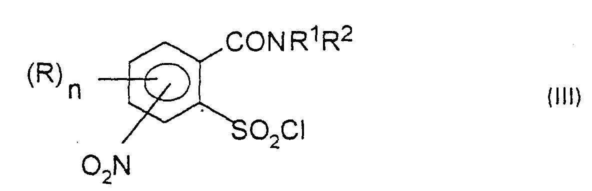
2. anschließend
a) die Verbindung (III) an der SO₂Cl-Gruppe zur Verbindung der Formel (IV) ammonolysiert, 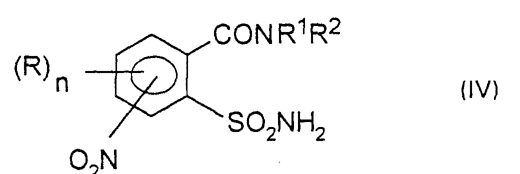 anschließend die Verbindung (IV) an der Nitrogruppe zur Verbindung (V) reduziert 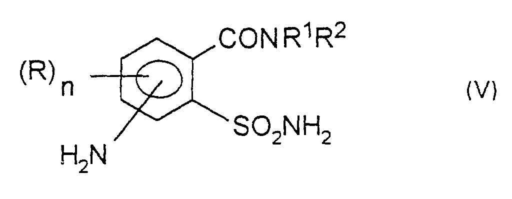 und anschließend die Verbindung (V) mit dem Carbamatsalz der Formel (VI), 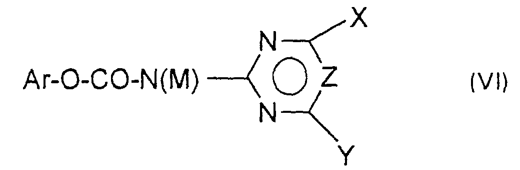 worin Ar ein unsubstituiertes oder substituiertes Phenyl darstellt und M ein Metallkation bedeutet, zur Verbindung der Formel (I) umsetzt, in der R³ in Übereinstimmung mit M in Formel (VI) H oder C₁-C₄-Alkyl oder im Falle M = Metallkation ein Wasserstoffatom bedeutet und A = H bedeutet, oder
b) die Verbindung der Formel (III) an der SO₂Cl-Gruppe zur genannten Verbindung der Formel (IV) ammonolysiert, anschließend die Verbindung (IV) mit dem Carbamat bzw. Carbamatsalz der genannten Formel (VI) zur Verbindung der Formel (VII) umsetzt, 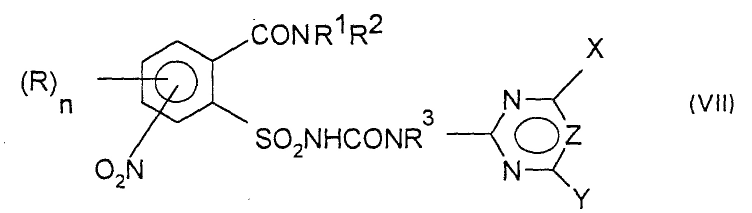 in der R³ wie M = H oder C₁-C₄-Alkyl analog Formel (VI) oder im Falle M = Metallkation ein Wasserstoffatom bedeutet, und die Verbindung der Formel (VII) an der Nitrogruppe zur Verbindung der Formel (I), worin A = H bedeutet, reduziert oder
c) die Verbindung der Formel (III) mit Cyanaten und dem heterocyclischen Amin der Formel (VIII), 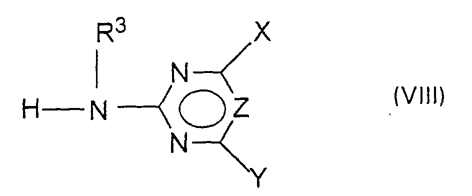
worin R³ wie in Formel (I) definiert ist, zum Sulfonylharnstoff der Formel (VII) umsetzt, welcher durch Reduktion an der Nitrogruppe die Verbindung der Formel (I), worin A = H bedeutet, ergibt, und
3. für den Fall, daß A im gewünschten Endprodukt der Formel (I) nicht Wasserstoff ist, sondern einen Acylrest bedeutet, die in Stufe 2 erhaltene Verbindung der Formel (I), worin A = H ist, acyliert,
wobei in den Formeln (II) bis (VIII) die Reste (R)ₙ, R¹, R², X, Y, Z wie im Endprodukt der Formel (I) definiert sind.

2. Verfahren nach Anspruch 1'; **dadurch gekennzeichnet, daß**
(R)ₙ n gleiche oder verschiedene Reste aus der Gruppe Halogen, C₁-C₄-Alkyl und C₁-C₄-Alkoxy,
n 0 oder 1,
A H oder Acyl mit 1 bis 8 C-Atomen,
R¹ H, C₁-C₆-Alkyl, C₂-C₆-Alkenyl, C₂-C₆-Alkinyl, C₁-C₆-Alkoxy, C₂-C₆-Alkenoxy, C₂-C₆-Alkinoxy oder C₅-C₆-Cycloalkyl, wobei jeder der 7 letztgenannten Reste unsubstituiert oder durch einen oder mehrere Reste aus der Gruppe Halogen, C₁-C₄-Alkoxy, C₁-C₄-Haloalkoxy, C₁-C₄-Alkylthio, Mono- und Di-(C₁-C₄-Alkyl)-amino, Cyano, Azido, Formyl, (C₁-C₄-Alkyl)-carbonyl, (C₁-C₄-Alkoxy)-carbonyl, C₁-C₄-Alkylsulfinyl und C₁-C₄-Alkylsulfonyl substituiert ist,
oder Phenyl, das unsubstituiert oder durch Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist,
R² H, C₁-C₆-Alkyl, C₂-C₆-Alkenyl oder C₂-C₆-Alkinyl, wobei jeder der drei letztgenannten Reste unsubstituiert oder durch einen oder mehrere Reste aus der Gruppe Halogen, C₁-C₄-Alkoxy, C₁-C₄-Haloalkoxy, C₁-C₄-Alkylthio, Mono- und Di-(C₁-C₄-Alkyl)-amino, Cyano, Azido, Formyl, (C₁-C₄-Alkyl)-carbonyl, (C₁-C₄-Alkoxy)-carbonyl, C₁-C₄-Alkylsulfinyl und C₁-C₄-Alkylsulfonyl substituiert ist,
oder die Gruppe
NR¹R² einen heterocyclischen Ring aus 4, 5 oder 6 Ringatomen, der bis zu zwei weitere Heteroringatome aus der Gruppe N und O im Ring enthalten kann und der unsubstituiert oder durch einen oder mehrere Reste aus der Gruppe C₁-C₄-Alkyl substituiert ist,
R³ H oder CH₃,
einer der Reste X und Y Halogen, C₁-C₂-Alkyl, C₁-C₂-Alkoxy, C₁-C₂-Alkylthio, wobei jeder der drei letztgenannten Reste unsubstituiert oder durch einen oder mehrere Reste aus der Gruppe Halogen, C₁-C₂-Alkoxy und C₁-C₂-Alkylthio substituiert ist, oder Mono- oder Di(C₁-C₂-alkyl)amino, und
der andere der Reste X und Y C₁-C₂-Alkyl, C₁-C₂-Haloalkyl, C₁-C₂-Alkoxy, C₁-C₂-Haloalkoxy oder C₁-C₂-Alkylthio,
Z CH oder N bedeuten.

3. Verfahren nach Anspruch 1 oder 2, **dadurch gekennzeichnet, daß**
A Wasserstoff, Formyl, (C₁-C₄)-Alkyl)-carbonyl, das unsubstituiert oder durch einen oder mehrere Reste aus der Gruppe Halogen und (C₁-C₄)-Alkoxy substituiert ist,
oder (C₁-C₄-Alkoxy)-carbonyl, Phenoxycarbonyl, Phenylcarbonyl, Phenyl-(C₁-C₄-alkyl)-carbonyl oder Phenyl-(C₁-C₂-alkoxy)-carbonyl, wobei Phenyl in jedem der letztgenannten 4 Reste unsubstituiert oder substituiert ist,
R¹ H, C₁-C₂-Alkyl, C₁-C₂-Alkoxy, Phenyl, das unsubstituiert oder durch einen oder mehrere Reste aus der Gruppe Halogen, C₁-C₂-Alkyl,
C₁-C₂-Alkoxy, C₁-C₂-Halogenalkyl und C₁-C₂-Halogenalkoxy substituiert ist,
R² H oder C₁-C₂-Alkyl,
oder die Gruppe
NR¹R² einen heterocyclischen Ring aus 5 oder 6 Ringatomen, der bis zu einem weiteren Heteroringatom aus der Gruppe N und O im Ring enthalten kann und der unsubstituiert oder durch einen oder mehrere C₁-C₂-Alkylreste substituiert ist,
R³ H oder CH₃ bedeuten.

4. Verfahren zur Herstellung einer Verbindung der Formel (III), 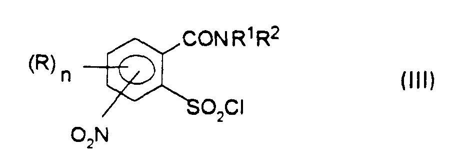 worin R, n, R¹ und R² wie in Formel (I) nach einem der Ansprüche 1 bis 3 definiert ist, **dadurch gekennzeichnet, daß** man die Verbindung der Formel (II), 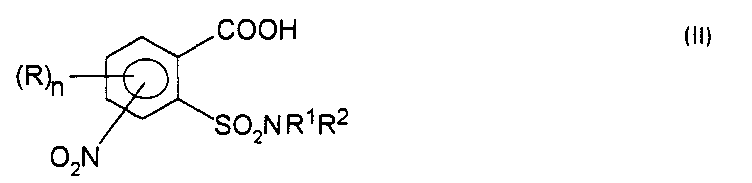 oder deren Salze, worin R, n, R¹ und R² wie in Formel (III) definiert ist, in Gegenwart eines Halogenierungsmittels unter Bildung des Carbonsäurehalogenids und dessen Umlagerung zur Verbindung der Formel (III) umsetzt.

5. Verfahren zur Herstellung einer Verbindung der Formel (I'), 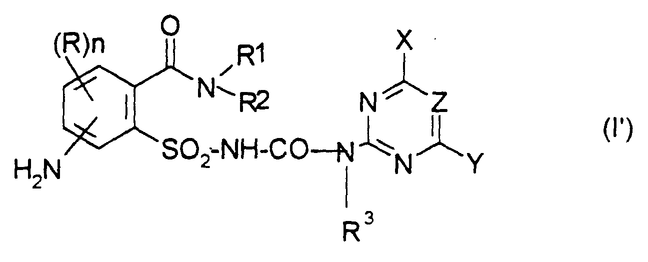 worin
R, R¹, R², X, Y, Z, n wie in Formel (I) nach einem der Ansprüche 1 bis 3 definiert sind und R³ = H bedeutet, **dadurch gekennzeichnet, daß** man die Verbindung der Formel (V), 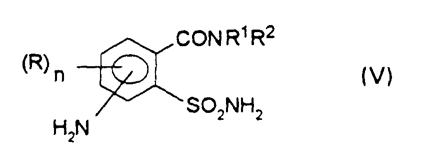 worin R, R¹, R², n wie in Formel (I) definiert sind, mit dem Carbamatsalz der Formel (VI), 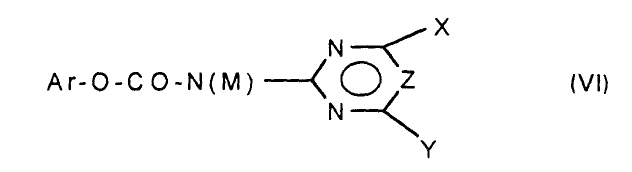 worin X, Y, Z, n wie in Formel (I) definiert sind, Ar ein unsubstituiertes oder substituiertes Phenyl darstellt und M ein Metallkation bedeutet, zur Verbindung der Formel (I') umsetzt.

6. Verfahren zur Herstellung von Verbindungen der Formel (I"), 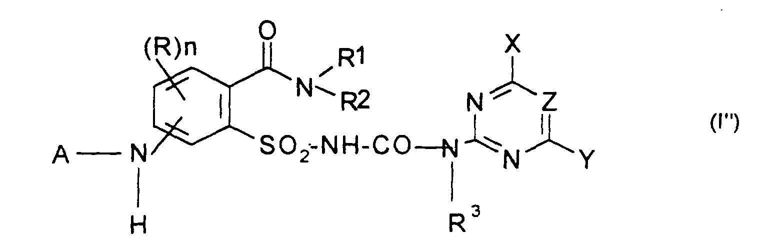 worin R, n, R¹, R², R³, X, Y, Z wie in Formel (I) nach einem der Ansprüche 1 bis 3 definiert sind und A einen Acylrest bedeutet, **dadurch gekennzeichnet, daß** man die Verbindung der Formel (I"), worin A = H ist, acyliert.

7. Verbindungen der Formel (I), wie sie in Anspruch 1 definiert sind und worin A = H ist.

8. Verbindungen der Formel (III), (IV) und (VII) wie sie in Anspruch 1 definiert sind.

9. Verbindungen der Formel (V), wie sie in Anspruch 1 definiert sind, ausgenommen die Verbindung der Formel (V), worin R¹=H, R²=H, die Aminogruppe NH₂ = eine Aminogruppe in Orthostellung zur Carbonamidgruppe der Formel CO-NR¹R² und (R)ₙ = ein Chloratom in Parastellung zur Carbonamidgruppe bedeuten.

10. Verbindungen der Formel (VI), wie sie in Anspruch 1 definiert sind und worin M ein Kation bedeutet.

## Claims

1. A process for preparing a compound of the formula (I) and salts thereof, 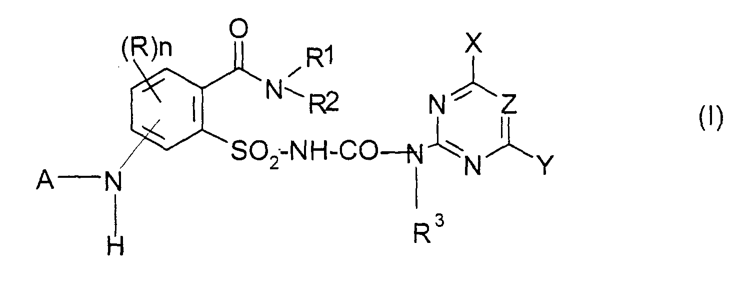 in which
(R)ₙ is n identical or different radicals from the group consisting of halogen, alkyl and alkoxy,
n is 0,1, 2 or 3,
A is hydrogen or an acyl radical,
R¹ is hydrogen or an unsubstituted or substituted hydrocarbon or hydrocarbonoxy radical having 1 to 6 carbon atoms,
R² is hydrogen or an unsubstituted or substituted hydrocarbon radical having a total of 1 to 10 carbon atoms,
or the group
NR¹R² is a heterocyclic ring having 3 to 8 ring atoms which is unsubstituted or substituted and contains the nitrogen atom of the group NR¹R² as ring heteroatom and may also contain one or two further ring heteroatoms from the group consisting of N, O and S,
R³ is hydrogen or C₁-C₄-alkyl,
X and Y independently of one another are halogen, C₁-C₆-alkyl, C₁-C₆-alkoxy, C₁-C₆-alkylthio, each of the three latter radicals being unsubstituted or substituted by one or more radicals from the group consisting of halogen, C₁-C₄-alkoxy and C₁-C₄-alkylthio, or are C₃-C₆-cycloalkyl, C₂-C₆-alkenyl, C₂-C₆-alkynyl, C₃-C₆-alkenyloxy or C₃-C₆-alkynyloxy, and
Z is CH or N,
which comprises
1. reacting the compound of the formula (II) 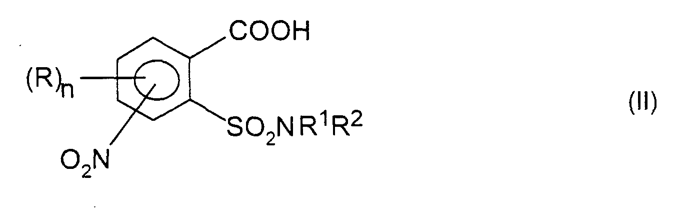 or its salts in an aprotic solvent in the presence of a halogenating agent, with formation of the carbonyl halide and its rearrangement to form the compound of the formula (III) 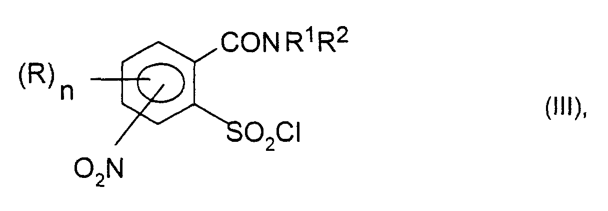
2. then
a) subjecting the compound (III) to ammonolysis at the SO₂Cl group to give the compound of the formula (IV) 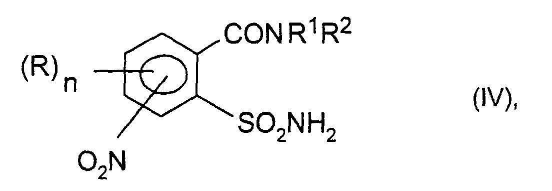 then reducing the compound (IV) at the nitro group to give the compound (V) 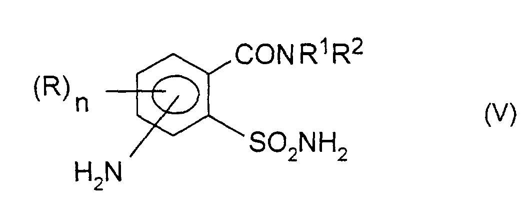 and then reacting the compound (V) with the carbamate salt of the formula (VI) 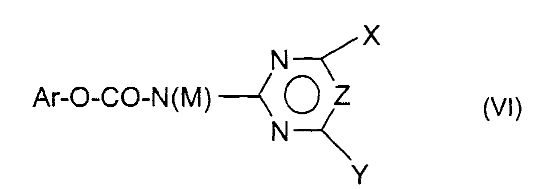 in which Ar is unsubstituted or substituted phenyl and M is a metal cation, to give the compound of the formula (I) in which R³ is, in agreement with M in formula (VI), a hydrogen atom, and A = H, or
b) subjecting the compound of the formula (III) to ammonolysis at the SO₂Cl group to give the abovementioned compound of the formula (IV), then reacting the compound (IV) with the carbamate salt of the abovementioned formula (VI) to give the compound of the formula (VII) 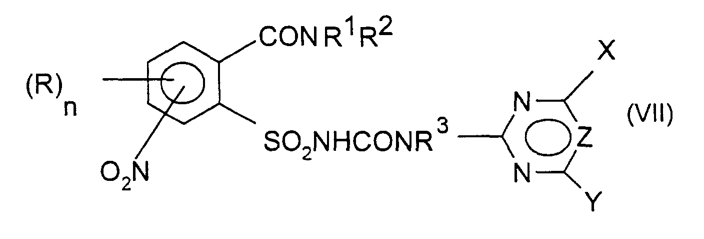 in which R³ is, like M, analogously to formula (VI), a hydrogen atom, and reducing the compound of the formula (VII) at the nitro group to give the compound of the formula (I) in which A is H, or
c) reacting the compound of the formula (III) with cyanates and with the heterocyclic amine of the formula (VIII) 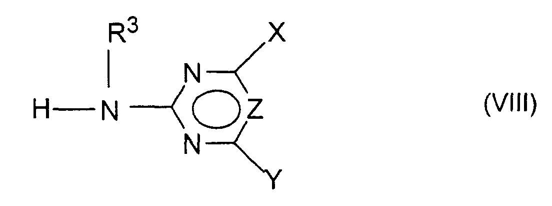 in which R³ is as defined in formula (I) to give the sulfonylurea of the formula (VII), which by reduction at the nitro group gives the compound of the formula (I) in which A is H, and
3. if A in the desired end product of the formula (I) is not hydrogen but is an acyl radical, acylating the compound of the formula (I) obtained in Stage 2, in which A is H,
the radicals (R)ₙ, R¹, R², X, Y, and Z in the formulae (II) to (VIII) being as defined for the end product of the formula (I).

2. A process as claimed in claim 1, wherein
(R)ₙ is n identical or different radicals from the group consisting of halogen, C₁-C₄-alkyl and C₁-C₄-alkoxy,
n is 0 or 1,
A is H or acyl having 1 to 8 carbon atoms,
R¹ is H, C₁-C₆-alkyl, C₂-C₆-alkenyl, C₂-C₆-alkynyl, C₁-C₆-alkoxy, C₂-C₆-alkenoxy, C₂-C₆-alkynoxy or C₅-C₆-cycloalkyl, each of the 7 latter radicals being unsubstituted or substituted by one or more radicals from the group consisting of halogen, C₁-C₄-alkoxy, C₁-C₄-haloalkoxy, C₁-C₄-alkylthio, mono- and di-(C₁-C₄-alkyl)-amino, cyano, azido, formyl, (C₁-C₄-alkyl)-carbonyl, (C₁-C₄-alkoxy)-carbonyl, C₁-C₄-alkylsulfinyl and C₁-C₄-alkylsulfonyl,
or is phenyl which is unsubstituted or substituted by radicals from the group consisting of halogen, C₁-C₄-alkyl, C₁-C₄-alkoxy, C₁-C₄-haloalkyl, C₁-C₄-haloalkoxy and nitro,
R² is H, C₁-C₆-alkyl, C₂-C₆-alkenyl or C₂-C₆-alkynyl, each of the three latter radicals being unsubstituted or substituted by one or more radicals from the group consisting of halogen, C₁-C₄-alkoxy, C₁-C₄-haloalkoxy, C₁-C₄-alkylthio, mono- and di-(C₁-C₄-alkyl)-amino, cyano, azido, formyl, (C₁-C₄-alkyl)-carbonyl, (C₁-C₄-alkoxy)-carbonyl, C₁-C₄-alkylsulfinyl and C₁-C₄-alkylsulfonyl,
or the group
NR¹R² is a heterocyclic ring of 4, 5 or 6 ring atoms which may contain up to two further ring heteroatoms from the group consisting of N and O in the ring and which is unsubstituted or substituted by one or more radicals from the group consisting of C₁-C₄-alkyl,
R³ is H or CH₃,
one of the radicals X and Y is halogen, C₁-C₂-alkyl, C₁-C₂-alkoxy, C₁-C₂-alkylthio, each of the three latter radicals being unsubstituted or substituted by one or more radicals from the group consisting of halogen, C₁-C₂-alkoxy and C₁-C₂-alkylthio, or is mono- or di-(C₁-C₂-alkyl)amino,
and
the other of the radicals X and Y is C₁-C₂-alkyl, C₁-C₂-haloalkyl, C₁-C₂-alkoxy, C₁-C₂-haloalkoxy or C₁-C₂-alkylthio,
Z is CH or N.

3. A process as claimed in claim 1 or 2, wherein
A is hydrogen, formyl, (C₁-C₄-alkyl)-carbonyl, which is unsubstituted or substituted by one or more radicals from the group consisting of halogen and (C₁-C₄)-alkoxy,
or is (C₁-C₄-alkoxy)-carbonyl, phenoxycarbonyl, phenylcarbonyl, phenyl-(C₁-C₄-alkyl)-carbonyl or phenyl-(C₁-C₂-alkoxy)-carbonyl, where the phenyl in each of the 4 latter radicals is unsubstituted or substituted,
R¹ is H, C₁-C₂-alkyl, C₁-C₂-alkoxy, phenyl which is unsubstituted or substituted by one or more radicals from the group consisting of halogen, C₁-C₂-alkyl, C₁-C₂-alkoxy, C₁-C₂-haloalkyl and C₁-C₂-haloalkoxy,
R² is H or C₁-C₂-alkyl,
or the group
NR¹R² is a heterocyclic ring of 5 or 6 ring atoms which may contain up to one further ring heteroatom from the group consisting of N and O in the ring and which is unsubstituted or substituted by one or more C₁-C₂-alkyl radicals,
R³ is H or CH₃.

4. A process for preparing a compound of the formula (III), 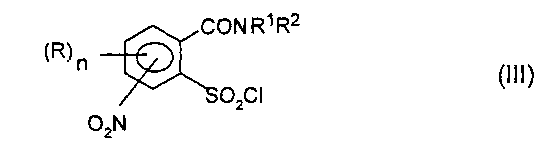 in which R, n, R¹ and R² are as defined for formula (I) as claimed in any one of claims 1 to 3, which comprises reacting the compound of the formula (II), 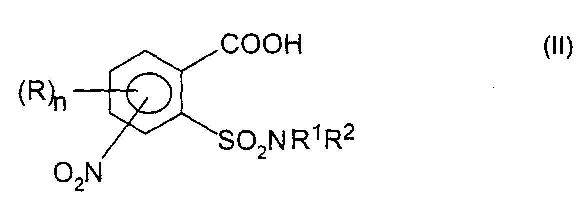 or a salt thereof, in which R, n, R¹ and R² are as defined in formula (III), in the presence of a halogenating agent, with the formation of the carbonyl halide and its rearrangement to form the compound of the formula (III).

5. A process for preparing a compound of the formula (I'), 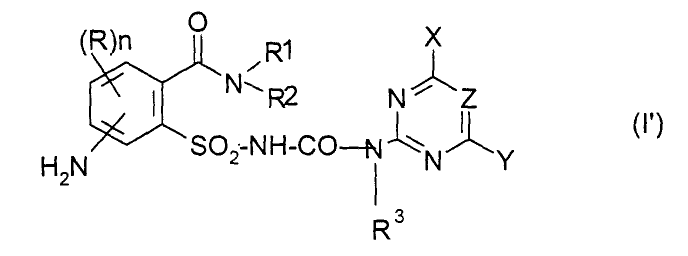 in which
R, R¹, R², X, Y, Z and n are as defined for formula (I) as claimed in any one of claims 1 to 3 and R³ is H, which comprises reacting the compound of the formula (V) 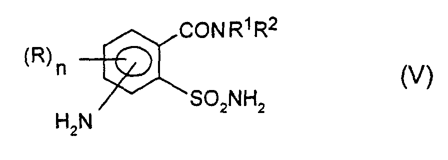 in which R, R¹, R² and n are as defined for formula (I) with the carbamate salt of the formula (VI) 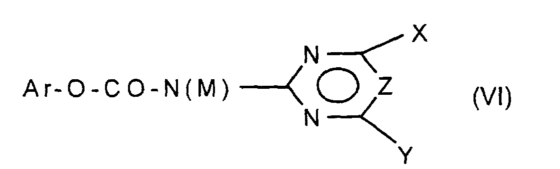 in which X, Y, Z and n are as defined for formula (I), Ar is an unsubstituted or substituted phenyl and M is a metal cation, to give the compound of the formula (I').

6. A process for preparing compounds of the formula (I") 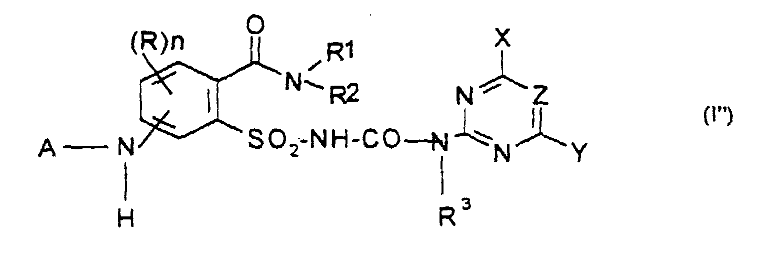 in which R, n, R¹, R², R³, X, Y and Z are as defined for formula (I) as claimed in any one of claims 1 to 3 and A is an acyl radical, which comprises acylating the compound of the formula (I") in which A is H.

7. A compound of the formula (I) as defined in claim 1, in which A is H.

8. A compound of the formula (III), (IV) or (VII) as defined in claim 1.

9. A compound of the formula (V) as defined in claim 1, with the exception of the compound of the formula (V) in which R¹=H, R²=H, the amino group NH₂ = an amino group positioned ortho to the carboxamido group of the formula CO-NR¹R² and (R)ₙ = a chlorine atom positioned para to the carboxamido group.

10. A compound of the formula (VI) as defined in claim 1, wherein M is a cation.

## Revendications

1. Procédé pour la préparation d'un composé de formule (I) et de ses sels, 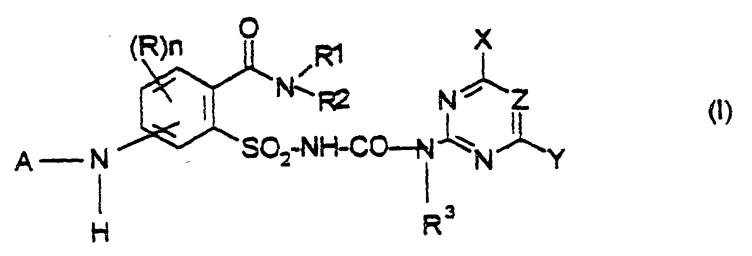 dans laquelle
(R)ₙ représente n groupes identiques ou différents choisis parmi un atome d'halogène, un groupe alkyle et alcoxy,
n représente 0, 1, 2 ou 3,
A représente un atome d'hydrogène ou un groupe acyle,
R¹ représente un atome d'hydrogène ou un groupe hydrocarbure ou oxyde d'hydrocarbure ayant de 1 à 6 atomes de carbone,
R² représente un atome d'hydrogène ou un résidu hydrocarboné non substitué ou substitué ayant en tout de 1 à 10 atomes de carbone,
ou le groupe
NR¹R² représente un cycle hétérocyclique ayant de 3 à 8 atomes de cycle, qui est non substitué ou substitué et contient l'atome d'azote du groupe NR¹R² comme hétéroatome de cycle et peut contenir encore un ou deux autres hétéroatomes de cycle choisis parmi N, O et S,
R³ représente un atome d'hydrogène ou un groupe alkyle en C₁ à C₄,
X, Y représentent indépendamment l'un de l'autre un atome d'halogène, un groupe alkyle en C₁ à C₆, alcoxy en C₁ à C₆, alkylthio en C₁ à C₆, dans lequel chacun des trois derniers groupes cités est non substitué ou substitué par un ou plusieurs groupes choisis parmi un atome d'halogène, un groupe alcoxy en C₁ à C₄ et alkylthio en C₁ à C₄, ou cycloalkyle en C₃ à C₆, alcényle en C₂ à C₆, alcynyle en C₂ à C₆, alcényloxy en C₃ à C₆ ou alcynyloxy en C₃ à C₆, et
Z représente CH ou N,
**caractérisé en ce que** l'on met à réagir
1. le composé de formule (II) 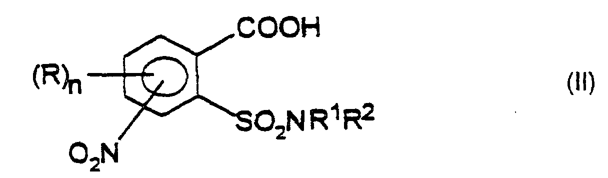 ou ses sels dans un solvant aprotique en présence d'un agent d'halogénation pour former l'halogénure d'acide carboxylique et son réarrangement en composé de formule (III) 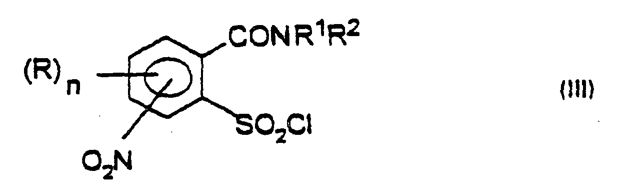
2. ensuite
a) on ammonolyse le composé (III) sur le groupe SO₂Cl pour obtenir le composé de formule (IV) 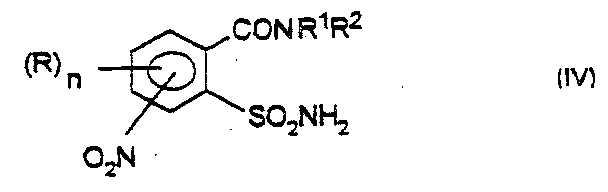 on réduit ensuite le composé (IV) sur le groupe nitro pour obtenir le composé (V) 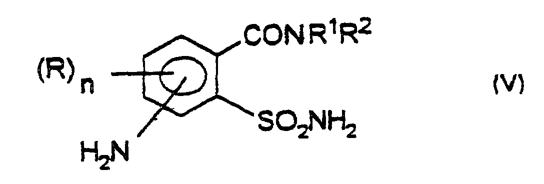 et ensuite on met à réagir le composé (V) avec le sel carbamate de formule (VI), 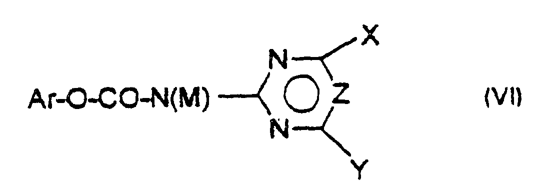 dans laquelle Ar représente un phényle non substitué ou substitué et M signifie un cation métallique, pour obtenir le composé de formule (I), dans laquelle R³ en accord avec M dans la formule (VI) représente H ou un groupe alkyle en C₁ à C₄ ou dans le cas où M = un cation métallique représente un atome d'hydrogène et A = H ou
b) on ammonolyse le composé de formule (III) sur le groupe SO₂Cl pour obtenir le composé cité de formule (IV), on met ensuite à réagir le composé (IV) avec le carbamate ou le sel de carbamate de formule (VI) citée pour obtenir le composé de formule (VII) 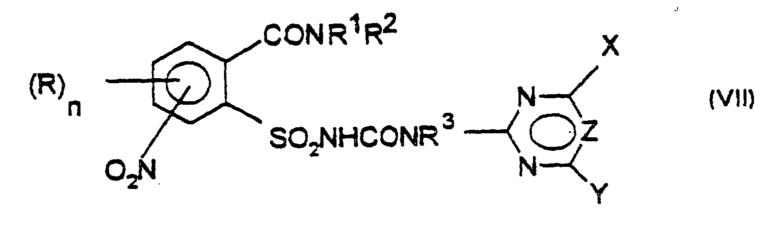 dans laquelle R³ signifie comme M = H ou un groupe alkyle en C₁ à C₄ de manière analogue à la formule (VI) ou dans le cas où M = un cation métallique représente un atome d'hydrogène, et on réduit le composé de formule (VII) sur le groupe nitro pour obtenir le composé de formule (I), dans laquelle A signifie H ou
c) on met à réagir le composé de formule (III) avec des cyanates et l'amine hétérocyclique de formule (VIII) 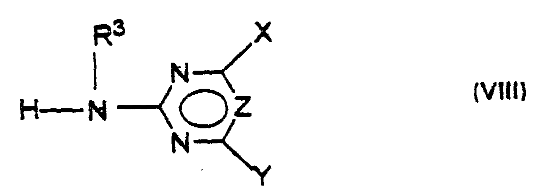 dans laquelle R³ est défini comme à la formule (I), pour obtenir la sulfonylurée de formule (VII), qui donne par réduction sur le groupe nitro le composé de formule (I), dans laquelle A = H, et
3. dans le cas où A n'est pas l'hydrogène dans le produit final souhaité de formule (I), mais représente au contraire un groupe acyle, on acyle le composé obtenu à l'étape 2 de formule (I), dans laquelle A = H,
les groupes (R)ₙ, R¹, R², X, Y, Z dans les formules (II) à (VIII) étant définis comme dans le produit final de formule (I).

2. Procédé selon la revendication 1, **caractérisé en ce que**
(R)ₙ représente n groupes identiques ou différents choisis parmi un atome d'halogène, un groupe alkyle en C₁ à C₄ et alcoxy en C₁ à C₄,
n représente 0 ou 1,
A représente H ou un groupe acyle ayant de 1 à 8 atomes de carbone,
R¹ représente H, un groupe alkyle en C₁ à C₆, alcényle en C₂ à C₆, alcynyle en C₂ à C₆, alcoxy en C₁ à C₆, alcénoxy en C₂ à C₆, alcynoxy en C₂ à C₆ ou cycloalkyle en C₅ à C₆, dans lesquels chacun des 7 derniers groupes cités est non substitué ou substitué par un ou plusieurs groupes choisis parmi un atome d'halogène, un groupe alcoxy en C₁ à C₄, halogénoalcoxy en C₁ à C₄, alkylthio en C₁ à C₄, mono- et di-(alkyle en C₁ à C₄)-amino, cyano, azido, formyle, (alkyle en C₁ à C₄)-carbonyle, (alcoxy en C₁ à C₄)-carbonyle, alkylsulfinyle en C₁ à C₄ et alkylsulfonyle en C₁ à C₄,
ou phényle, qui est non substitué ou substitué par des groupes choisis parmi un atome d'halogène, un groupe alkyle en C₁ à C₄, alcoxy en C₁ à C₄, halogénoalkyle en C₁ à C₄, halogénoalcoxy en C₁ à C₄, et nitro,
R² représente H, un groupe alkyle en C₁ à C₆, alcényle en C₂ à C₆, ou alcynyle en C₂ à C₆, dans lesquels chacun des trois derniers groupes cités est non substitué ou substitué par un ou plusieurs groupes choisis parmi un atome d'halogène, un groupe alcoxy en C₁ à C₄, halogénoalcoxy en C₁ à C₄, alkylthio en C₁ à C₄, mono- et di-(alkyle en C₁ à C₄)-amino, cyano, azido, formyle, (alkyle en C₁ à C₄)-carbonyle, (alcoxy en C₁ à C₄)-carbonyle, alkylsulfinyle en C₁ à C₄ et alkylsulfonyle en C₁ à C₄,
ou le groupe
NR¹R² représente un cycle hétérocyclique constitué de 4, 5 ou 6 atomes de cycle, qui peut contenir jusqu'à deux autres hétéroatomes de cycle choisis parmi N et O et qui est non substitué ou substitué par un ou plusieurs groupes choisis parmi un groupe alkyle en C₁ à C₄,
R³ représente H ou CH₃,
un des groupes X et Y représente un atome d'halogène, un groupe alkyle en C₁ à C₂, alcoxy en C₁ à C₂, alkylthio en C₁ à C₂, dans lesquels chacun des trois derniers groupes cités est non substitué ou substitué par un ou plusieurs groupes choisis parmi un atome d'halogène, un groupe alcoxy en C₁ à C₂ et alkylthio en C₁ à C₂, ou mono- ou di-(alkyle en C₁ à C₂) -amino, et
les autres des groupes X et Y représentent un groupe alkyle en C₁ à C₂, halogénoalkyle en C₁ à C₂, alcoxy en C₁ à C₂, halogénoalcoxy en C₁ à C₂ ou alkylthio en C₁ à C₂,
Z représente CH ou N.

3. Procédé selon la revendication 1 ou 2, **caractérisé en ce que**
A représente un atome d'hydrogène, un groupe formyle, (alkyle en C₁ à C₄)-carbonyle, qui est non substitué ou substitué par un ou plusieurs groupes choisis parmi un atome d'halogène et un groupe alcoxy en C₁ à C₄,
ou un groupe (alcoxy C₁ à C₄)-carbonyle, phénoxycarbonyle, phénylcarbonyle, phényl-(alkyle en C₁ à C₄)-carbonyle ou phényl-(alcoxy C₁ à C₂)-carbonyle, dans lesquels le phényle est dans chacun des quatre derniers groupes cités non substitué ou substitué,
R¹ représente H, un groupe alkyle en C₁ à C₂, alcoxy en C₁ à C₂, phényle, qui est non substitué ou substitué par un ou plusieurs groupes choisis parmi un atome d'halogène, un groupe alkyle en C₁ à C₂, alcoxy en C₁ à C₂, halogénoalkyle en C₁ à C₂ et halogénoalcoxy en C₁ à C₂,
R² représente H ou un groupe alkyle en C₁ à C₂,
ou le groupe
NR¹R² représente un cycle hétérocyclique constitué de 5 ou 6 atomes de cycle, qui peut contenir jusqu'à un autre hétéroatome de cycle choisi parmi le groupe N et O et qui est non substitué ou substitué par un ou plusieurs groupes alkyle en C₁ à C₂,
R³ représente H ou CH₃.

4. Procédé pour la préparation d'un composé de formule (III), 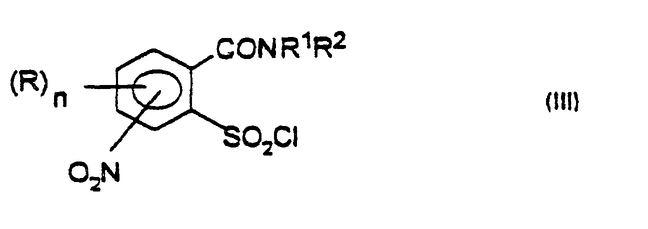 dans laquelle R, n, R¹ et R² sont définis comme à la formule (I) selon l'une des revendications 1 à 3, **caractérisé en ce que** l'on met à réagir le composé de formule (II) 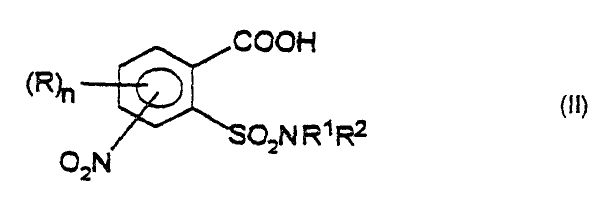 ou ses sels, dans laquelle R, n, R¹ et R² sont définis comme à la formule (III) en présente d'un agent d'halogénation en formant l'halogénure d'acide carboxylique et son réarrangement pour obtenir le composé de formule (III).

5. Procédé pour la préparation d'un composé de formule (I'), 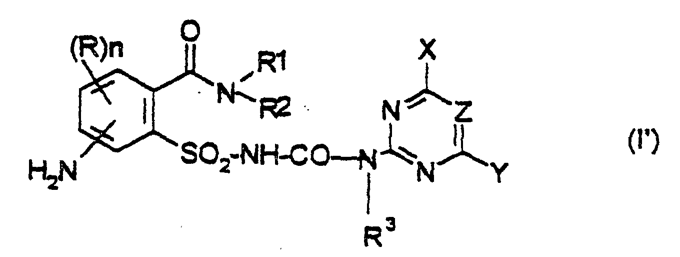 dans laquelle R, R¹, R², X, Y, Z, n sont définis comme à la formule (I) selon l'une des revendications 1 à 3 et R³ = H, **caractérisé en ce que** l'on met à réagir le composé de formule (V), 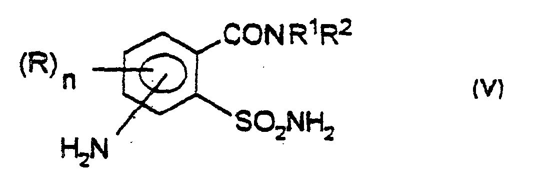 dans laquelle R, R¹, R², n sont définis comme à la formule (I), avec le sel carbamate de formule (VI) 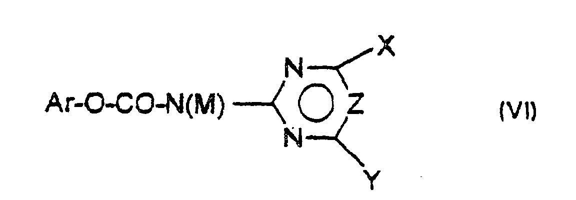 dans laquelle X, Y, Z, n sont définis comme à la formule (I), Ar représente un phényle non substitué ou substitué et M représente un cation métallique, pour obtenir le composé de formule (I').

6. Procédé pour la préparation de composés de formule (I"), 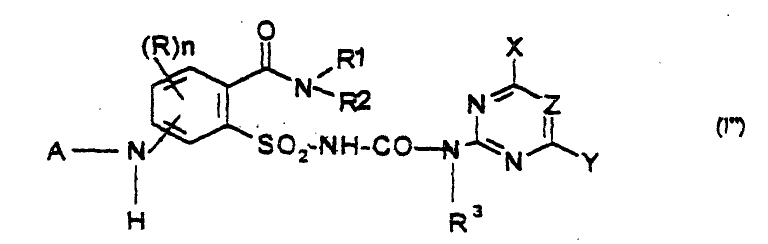 dans laquelle R, n, R¹, R², R³, X, Y, Z sont définis comme à la formule (I), selon l'une des revendications 1 à 3 et A représente un groupe acyle, **caractérisé en ce qu'**on acyle le composé de formule (I"), dans laquelle A = H.

7. Composés de formule (I), comme ils sont définis à la revendication 1 et dans laquelle A = H.

8. Composés de formules (III), (IV) et (VII) comme ils sont définis à la revendication 1.

9. Composés de formule (V), comme ils sont définis à la revendication 1, excepté le composé de formule (V), dans laquelle R¹ = H, R² = H, le groupe amino NH₂ = un groupe amino en position ortho par rapport au groupe carbonamide de formule CO-NR¹R² et (R)ₙ = un atome de chlore en position para par rapport au groupe carbonamide.

10. Composés de formule (VI), comme ils sont définis à la revendication 1 et dans lesquels M représente un cation.
